# EUROPEAN PATENT SPECIFICATION

(11) **EP 2 464 330 B1**
(45) Date of publication and mention of the grant of the patent: **23.03.2016**
(21) Application number: 09736580.3
(22) Date of filing: 29.09.2009
(51) Int. Cl.: A61K 9/00, A61Q 11/00, A61K 38/20, A61P 1/02

(54) **ORAL CARE COMPOSITIONS CONTAINING HUMAN RECOMBINANT INTERLEUKIN-1**
REKOMBINANTES HUMANES INTERLEUKIN-1 ENTHALTENDE ZUSAMMENSETZUNGEN ZUR MUNDPFLEGE
COMPOSITIONS DE SOIN BUCCAL CONTENANT DE L'INTERLEUKIN-1 HUMAINE

(43) Date of publication of application: 20.06.2012
(73) Proprietor: United Technologies UT AG, 8047 Zürich (CH)
(72) Inventor: PETROPAVLOV, Igor Arturovich, CH-8700 Küsnacht (CH); POMYTKIN, Igor Anatolievich, Moscow 123557 (RU)
(74) Representative: Boult Wade Tennant
(86) International application number: PCT/EP2009/062639
(87) International publication number: WO 2011/038754

(56) References cited:
- EP-A2- 0 352 816
- WO-A1-2005/089722
- WO-A1-2008/057136
- WO-A2-2008/008596
- US-A- 5 202 118
- US-A- 6 045 800
- US-A1- 2007 053 849
- PORTER S R ET AL: "Oral malodour (halitosis)" BMJ, vol. 333, no. 7569, September 2006 (2006-09), pages 632-635, XP002601188

## Description

### Field of the Invention

The invention relates to oral care compositions comprising human recombinant interleukin-1 for use in reducing oral malodor, and/or preventing or treating periodontal diseases.

### Background of the Invention

Interleukin-1 family of cytokines is represented by some members. Among them, interleukin-1 alpha and interleukin-1 beta are two major isoforms with amino acid sequences well-known from the art. Among them, only interleukin-1 alpha is a polypeptide synthesized and secreted by oral mucosal epithelial cells on a constitutive basis. Li J, Farthing PM, Ireland GW, Thornhill MH. IL-1 alpha and IL-6 production by oral and skin keratinocytes: similarities and differences in response to cytokine treatment in vitro. J Oral Pathol Med. 1996, 25(4): 157-62. As generally believed, interleukin-1 alpha plays a key role in the initiation of reparative processes following oral mucosa injury or bacterial infection.

Along with the positive role in the oral mucosa reparative processes, interleukin-1 is considered to be a strong pathogenic factor in the development of periodontal disease. This disease is caused by bacterial infection and is characterized by inflammatory destruction of tissues that support the teeth, including connective tissue and bone. Periodontal pathogenic bacteria induce elevated levels of interleukin-1. Gingival crevicular fluid levels of IL-1 are elevated in periodontitis, and decrease following treatment. Masada MP, Persson R, enney JS, Lee SW, Page RC, Allison AC. Measurement of interleukin-1 alpha and -1 beta in gingival crevicular fluid: implications for the pathogenesis of periodontal disease. J Periodontal Res 1990, 25 : 156-163. Tissue levels of IL-1 beta are elevated at sites of progressive periodontitis. Stashenko P, Fujiyoshi P, Obernesser MS, Prostak L, Haffajee AD, Socransky SS (1991). Levels of interleukin-1 beta in tissue from sites of active periodontal disease. J Clin Periodontol 18:548-554. Figueredo CM, Ribeiro MS, Fischer RG, Gustafsson A. Increased interleukin-1 beta concentration in gingival crevicular fluid as a characteristic of periodontitis. J Periodontol 1999;70: 1457-1463. Zhong Y, Slade GD, Beck JD, Offenbacher S. Gingival crevicular fluid interleukin-1 beta, prostaglandin E2 and periodontal status in a community population. J Clin Periodontol 2007:34:285-293. Elevated levels of IL-1 alpha in the oral micro-environment of transgenic mice that overexpress IL-1 alpha in the basal layer of oral mucosal epithelium can mediate all of the clinical features of periodontal disease. Dayan S, Stashenko P, Niederman R, upper TS. Oral epithelial overexpression of IL-1 alpha causes periodontal disease. J Dent Res. 2004, 83(10):786-90. Thus, interleukin-1 is considered to be a strong pathogenic factor of periodontal disease. Nothing has been published or disclosed in the art related to oral care compositions comprising interleukin-1 for the treatment or prevention of periodontal diseases.

Oral malodor, also known as halitosis or bad breath, is a significant social and/or psychological problem. Oral malodor is most common attributed to microbial populations that cause gingivitis and periodontitis. McNamara TF, Alexander JF and Lee M, The role of microorganisms in the production of oral malodor. Oral Surg Oral Med Oral Pathol 34(1):41-8. 1972. Kostelc JG, Preti G et al. Oral odors in early experimental gingivitis. J Periodontal Res 19(3):303-12, 1984. Yaegaki K and Sanada K, Biochemical and clinical factors influencing oral malodor in periodontal patients. J Periodontol 63(9):783-9, 1992. Amino acids, peptides, and proteins in the oral cavity are metabolized by bacteria in oral cavity to form malodorous volatile compounds. Examples of such compounds include hydrogen sulfide, methyl mercaptan, and dimethyl sulfide (formed from the sulfur containing amino acids); indole and skatole (formed from tryptophan); cadaverine and putrescine (formed from lysine and ornithine); and butyrate and valerate (formed from the other amino acids). The treatment of oral malodour can therefore be focused on the reduction of the intraoral bacterial load. Quirynen M, Zhao H, van Steenberghe D. Review of the treatment strategies for oral malodour. Clin Oral Invest. 2002, 6: 1-10. Nothing has been published or disclosed in the art related to oral care compositions comprising interleukin-1 for the treatment or prevention of oral malodor.

Use of interleukin-1 in medicinal applications is known from the art. For example, U.S. Patent No. 4,816,436 discloses a process for treating arthritis or inflammation with the use of intra-articular, intramuscular, intravenous, or intraperitoneal injections of interleukin-1 alpha; U.S. Patent No. 5,120,534 discloses a method for treating thrombocytopenia by administering interleukin-1 alpha or Asp36, Ser141-derivative of interleukin-1 alpha; U.S. Patent No. 5,534,251 discloses stabilized medicinal composition comprising Asp36, Ser141-derivative of interleukin-1 alpha; EP0391444 discloses a pharmaceutical composition comprising interleukin-1 alpha, and suitable for forming a parenterally administratable aqueous formulation; WO9116916, JP4018033, EP0482213, and ES2121782T disclose an antitumor composition containing the combination of interleukin-1 and gamma- interferon. However, no published or disclosed in the art related to oral care products comprising interleukin-1, including human recombinant interleukin-1 alpha and beta.

Human interleukin-1 may be prepared in industrial scale and suitable purity as recombinant polypeptides identical to the native human polypeptides. Gubler U. et al. Recombinant human interleukin-1 alpha: purification and biological characterization. J. Immunol. 1986, 136:2492-2497. U.S. Pat. No. 6,268,180 discloses a method of preparation of human recombinant interleukin-1 alpha.

Surprisingly, we found that compositions of the human recombinant interleukin-1 is useful for keeping oral cavity in a good condition, reducing malodor, and preventing and treating periodontal diseases.

It is an object of the present invention to provide oral care compositions comprising human recombinant interleukin-1 and methods thereof for keeping oral cavity in a good condition, reducing oral malodor, and preventing or treating periodontal diseases. United States Patent 5,202,118 describes compositions comprising interleukin-1 for use in promoting wound healing.

### Detailed Description of the Invention

The present invention provides an oral care composition comprising a human recombinant interleukin-1 and an orally acceptable carrier for use in reducing oral malodor in a subject in need thereof. Also provided is an oral care composition comprising a human recombinant interleukin-1 and an orally acceptable carrier for use in the treatment or prevention of a periodontal disease.

As used herein, the term "human recombinant interleukin-1" refers to polypeptide, which is expressed using suitable recombinant protein expression systems that use, for example, *E. coli* or yeast as the host. Preparation of human recombinant interleukin-1 is described, for example, in J. Immunol. 1986, 136(7):2429 and in U.S. Pat. No. 6,268,180.

In preferred embodiments of the present invention, the human recombinant interleukin-1 is a human recombinant interleukin-1 alpha or a human recombinant interleukin-1 beta.

As used herein, the term "human recombinant interleukin-1 alpha" refers to a polypeptide, which is expressed using suitable recombinant protein expression systems that use, for example, *E. coli* or yeast as the host, and which has the amino acid sequence of the human interleukin-1 alpha, or its biologically active analogues, or derivatives thereof.

As used herein, the term "the human interleukin-1 alpha" refers to the polypeptide well-known from the art, published, for example, in UniProt database No. P01583 (http://www.uniprot.org/uniprot/P01583), and having the following amino acid sequence:

SAPFSFLSNVKYNFMRIIKYEFILNDALNQSIIRANDQYLTAAALHNLDEAVKFDM
GAYKSSKDDAKITVILRISKTQLYVTAQDEDQPVLLKEMPEIPKTITGSETNLLFF
WETHGTKNYFTSVAHPNLFIATKQDYWVCLAGGPPSITDFQI LENQA

As used herein, the term "analogue of human interleukin-1 alpha" refers to an interleukin-1 alpha that contains one or more amino acid substitutions, deletions, additions, or rearrangements compared with human interleukin-1 alpha at sites such that the interleukin-1 alpha analogue still retains the in vitro and/or vivo biological activity of the human interleukin-1 alpha. Examples of such analogues are described in U.S. Pat. No. 6,268,180 and U.S. Pat.No. 5,120,534.

As used herein, the term "derivative of human interleukin-1 alpha" refers to human interleukin-1 alpha and the human interleukin-1 alpha analogues that are chemically or enzymatically derivatized at one or more constituent amino acids, including side chain modifications, backbone modifications, and N-and C-terminal modifications, by for example acetylation, acylation, hydroxylation, methylation, amidation, phosphorylation, pegylation, or glycosylation, and that retain the in vivo biological activity of interleukin-1 alpha. An example of a human interleukin-1 alpha derivative is N6-myristoyl-Lysl 1 - interleukin-1 alpha and HisTag-interleukin-1 alpha.

As used herein, the term "human recombinant interleukin-1 beta" refers to polypeptide, which is expressed using suitable recombinant protein expression systems that use, for example, E-coli or yeast as the host, and has the amino acid sequence of human interleukin-1 beta, or its biologically active analogues, or derivatives thereof.

As used herein, the term "the human interleukin-1 beta" refers to the polypeptide well-known from the art, published, for example, in UniProt database No. P01584 (http://www.uniprot.org/uniprot/P01584), and having the following amino acid sequence:

APVRSLNCTLRDSQQKSLVMSGPYELKALHLQGQDMEQQVVFSMSFVQGEESN
DKIPVALGLKEKNLYLSCVLKDDKPTLQLESVDPKNYPKKKMEKRFVFNKIEINN
KLEFESAQFPNWYISTSQAENMPVFLGGTKGGQDITDFTMQFVSS

As used herein, the term "analogue of human interleukin-1 beta" refers to an interleukin-1 beta that contains one or more amino acid substitutions, deletions, additions, or rearrangements compared with human interleukin-1 beta at sites such that the interleukin-1 beta analogue still retains the in vivo biological activity of interleukin-1 beta. Examples of interleukin-1 beta analogues include OCT-43 (Otsuka Pharmaceutical Co., Ltd., Tokyo, Japan) and others described in U.S. Pat. No. 6,107,465 and U.S. Pat. No. 5,847,098.

As used herein, the term "derivative of human interleukin-1 beta" refers to human interleukin-1 beta and human interleukin-1 beta analogues that are chemically or enzymatically derivatized at one or more constituent amino acids, including side chain modifications, backbone modifications, and N-and C-terminal modifications, by for example acetylation, acylation, hydroxylation, methylation, amidation, phosphorylation, pegylation, or glycosylation, and that retain the in vivo biological activity of interleukin-1 beta. An example of an interleukin-1 beta derivative is myristoyl-Lys16-interleukin-1 beta and HisTag-interleukin-1 beta.

In preferred embodiments of the present invention, the content of the human recombinant interleukin-1 in compositions for use according to the present invention is from 10⁻⁷ to 10⁻⁴ wt. %.

In preferred embodiments of the present invention, compositions for use according to the present invention further comprise a buffer at a concentration effective to maintain the pH of the composition at between about 4.0 to about 10.0. Examples of orally acceptable buffers include, but are not limited to, phosphate buffer, acetate, citrate buffer, succinate buffer, and glycine buffer.

As used herein, the term "oral care composition" refers to any composition suitable for administration to the oral cavity of a human or animal subject for enhancing the health, hygiene or appearance of the subject, preferably providing such benefits as: the prevention or treatment of a condition or disorder of the teeth, gums, mucosa or other hard or soft tissue of the oral cavity; the prevention or treatment of a systemic condition or disorder; and combinations thereof. In various embodiments, an oral care composition is not intentionally swallowed for purposes of systemic administration of components of the composition, but is rather retained in the oral cavity for a time sufficient to contact substantially all of oral tissues for purposes of oral activity. The oral composition for use according to the present invention may be in the form of a toothpaste, tooth gel, subgingival gel, dentifrice, tooth powder, mouthrinse, denture product, mouthspray, oral tablet, or chewing gum. The oral composition may also be incorporated onto strips or films for the application or attachment to oral surfaces.

As used herein, the term "orally acceptable carrier" refers to one or more safe solid or liquid diluents or encapsulating substances compatible with the human recombinant interleukin-1 and are suitable for topical oral administration. The term "compatible", as used herein, means the substance capable of being mixed with the human recombinant interleukin-1 without interaction in a manner which would substantially reduce the interleukin-1's stability and/or efficacy. Non-exclusive examples of such orally acceptable carriers include distilled or deionized water, calcium carbonate, calcium citrate, bentonite, and montmorillonite.

The compositions for use according to the present invention can comprise optional ingredients. Such optional ingredients generally are used individually at levels from about 0.0005% to about 10.0%, preferably from about 0.005% to about 1.0% by weight of the composition.

Examples of suitable optional ingredients include, but are not limited to, fluoride ion sources, alkali metal bicarbonate sources, humectants, anticalculus agents, abrasive polishing materials, thickening materials, surfactants, titanium dioxide, flavoring and sweetening agents, xylitol, coloring agents, teeth whitening agents, bentonite, montmorillonite, other active ingredients, and mixtures thereof.

Examples of suitable fluoride ion sources include, but are not limited to, sodium fluoride, potassium fluoride, sodium monofluorophosphate.

Examples of suitable alkali metal bicarbonate sources include, but are not limited to, sodium bicarbonate, and potassium bicarbonate.

Examples of suitable humectants include, but are not limited to, water soluble liquid polyols selected from the group comprising glycerine, propylene glycol, sorbitol, xylitol, butylene glycol, polyethylene glycol, and mixtures thereof.

Examples of suitable anticalculus agents include, but are not limited to, synthetic anionic polymers, including polyacrylates and copolymers of maleic anhydride or acid and methyl vinyl ether (e.g. Gantrez).

Examples of suitable abrasive polishing materials include, but are not limited to, silica, hydrated alumina, calcium carbonate, calcium citrate, dicalcium orthophosphate dihydrate, calcium pyrophosphate, tricalcium phosphate, calcium polymetaphosphate, and resinous abrasive materials.

Examples of suitable thickening materials include, but are not limited to, carboxyvinyl polymers, carrageenan, hydroxyethyl cellulose, water soluble salts of cellulose ethers such as sodium carboxymethylcellulose and sodium carboxymethyl hydroxyethyl cellulose, natural gums such as gum karaya, xanthan gum, gum arabic, and gum tragacanth, and mixtures thereof.

Examples of suitable surfactants include, but are not limited to, sodium and potassium salts of the following: lauroyl sarcosinate, myristoyl sarcosinate, palmitoyl sarcosinate, stearoyl sarcosinate and oleoyl sarcosinate.

Examples of suitable teeth whitening agents include, but are not limited to, talc, mica, magnesium carbonate, calcium carbonate, magnesium silicate, aluminum magnesium silicate, silica, titanium dioxide, zinc oxide, red iron oxide, brown iron oxide, yellow iron oxide, black iron oxide, ferric ammonium ferrocyanide, manganese violet, ultramarine, nylon powder, polyethylene powder, methacrylate powder, polystyrene powder, silk powder, crystalline cellulose, starch, titanated mica, iron oxide titanated mica, bismuth oxychloride, and mixtures thereof. Pigments that are generally recognized as safe, and are listed in C.T.F.A. Cosmetic Ingredient Handbook, 3rd Ed., Cosmetic and Fragrance Assn., Inc., Washington D.C. (1982).

Examples of suitable flavoring agents include, but are not limited to, oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and mixtures thereof.

Examples of suitable sweetening agents include, but are not limited to, sucrose, glucose, saccharin, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate and sodium saccharin, and mixtures thereof.

Examples of suitable other active ingredients include, but are not limited to, antimicrobial agents, enzymes, and antioxidants.

Examples of suitable antimicrobial agents include, but are not limited to, phenol and its homologs, mono and poly-alkyl and aromatic halophenols, resorcinol and its derivatives, bisphenolic compounds and halogenated salicylanilides, benzoic esters, and halogenated phenols, quaternary ammonium agents, copper bisglycinate, copper glycinate, zinc citrate, zinc lactate, chlorhexidine, triclosan, triclosan monophosphate, and flavor oils such as thymol.

Examples of suitable enzymes include, but are not limited to, proteases including papain, pepsin, trypsin, ficin, bromelin; cell wall lytic enzymes including lysozyme; plaque matrix inhibitors including dextranases, mutanases; and oxidases including glucose oxidase, lactate oxidase, galactose oxidase, uric acid oxidase, peroxidases including horse radish peroxidase, myeloperoxidase, lactoperoxidase, and chloroperoxidase.

The compositions for use according to the invention are prepared by standard techniques well known to those skilled in the art. Such procedures include, but are not limited to, mixing the human recombinant interleukin-1 with other ingredients of the composition in conventional manner.

Further, described herein is use of a human recombinant interleukin-1 for the manufacturing a composition for oral care.

In preferred embodiments of the present invention, the human recombinant interleukin-1 is human recombinant interleukin-1 alpha.

In preferred embodiments of the present invention, the human recombinant interleukin-1 is human recombinant interleukin-1 beta.

In preferred embodiments of the present invention, the compositions are useful for keeping the oral tissues and/or the dental surfaces in a good condition, for preventing or treating oral malodor, and preventing or treating periodontal diseases. Such periodontal diseases include, but are not limited to, gingival diseases, chronic periodontitis, aggressive periodontitis, periodontitis as manifestation of systemic diseases, necrotizing periodontal disease, periodontal abscess, periodontitis with endodontic lesion, and developed and acquired deformation and conditions as classified in current classification of periodontal diseases according, for example, to the 1999 International Workshop for Classification of Periodontal Diseases and Conditions in Oak Brook (Illinois, USA), 30.10.1999 to 2.11.1999. Ann Periodontol 1999, 4: 1-6.

Further, the present invention provides an oral care composition comprising a human recombinant interleukin-1 and an orally acceptable carrier for reducing oral malodor comprising a step of applying to the surface of the oral cavity of a subject in need thereof.

Further, the present invention provides an oral care composition comprising a human recombinant interleukin-1 and an orally acceptable carrier for the treatment or prevention of a periodontal disease comprising a step of applying to the surface of the oral cavity of a subject in need thereof an oral care composition comprising a human recombinant interleukin-1 and an orally acceptable carrier.

In preferred embodiments of the present invention, the periodontal disease is periodontitis and/or gingival disease.

In practicing the present invention, a safe and effective amount of the compositions of the present invention may be topically applied to the mucosal tissue of the oral cavity, to the gingival tissue of the oral cavity, and/or to the surface of the teeth, for the treatment or prevention of the above mentioned diseases or conditions of the oral cavity, preferably for at least about from 0.1 to about 10 minutes, more preferably from 0.5 to 1 minute in several conventional ways. For example, the gingival or mucosal tissue may be rinsed with a solution (e.g. mouth rinse, mouth spray) containing human recombinant interleukin-1; or if human recombinant interleukin-1 is included in a dentifrice (e.g. toothpaste, tooth gel or tooth powder), the gingival/mucosal tissue or teeth is bathed in the liquid and/or lather generated by brushing the teeth. Other non-limiting examples include applying a non-abrasive gel or paste, which contains human recombinant interleukin-1, directly to the gingival/mucosal tissue or to the teeth with or without an oral care appliance described below; chewing gum that contains human recombinant interleukin-1; chewing or sucking on a breath tablet or lozenge which contains human recombinant interleukin-1. This method can be reapplied from 1 to about 5, preferably from 1 to 2 times per day. Typically, the effective amount of the composition is from about 0.5 to about 10 grams, preferably about 1 gram.

The following examples are presented to demonstrate the invention. The examples are illustrative only and are not intended to limit the scope of the invention in any way.

### Example 1

Toothpaste containing human recombinant interleukin-1 alpha as active ingredient

| Ingredient | Content, wt.% |
|---|---|
| Human recombinant Interleukin 1 alpha | 0.00001 |
| Calcium citrate | 3 |
| Calcium hydrogen phosphate | 37 |
| Glycerol | 10 |
| Sorbitol | 10 |
| Carboxymethylcellulose | 1 |
| Sodium laurylsulfate | 2 |
| Flavor | 1 |
| Phosphate buffer | qs to pH 5.5 |
| Distilled water | to 100 |

The abovementioned ingredients were mixed and kneaded in the conventional manner to prepare toothpaste containing human recombinant interleukin-1 alpha.

### Example 2

Toothpaste containing human recombinant interleukin-1 beta as active ingredient

| Ingredient | Content, wt.% |
|---|---|
| Human recombinant Interleukin 1 beta | 0.00001 |
| Calcium citrate | 3 |
| Calcium hydrogen phosphate | 37 |
| Glycerol | 10 |
| Sorbitol | 10 |
| Carboxymethylcellulose | 1 |
| Sodium laurylsulfate | 2 |
| Flavor | 1 |
| Phosphate buffer | qs to pH 5.5 |
| Distilled water | to 100 |

The abovementioned ingredients were mixed and kneaded in the conventional manner to prepare toothpaste containing human recombinant interleukin-1 beta.

### Example 3

Topical oral gel containing human recombinant interleukin-1 alpha as active ingredient

| Ingredient | Content, wt.% |
|---|---|
| Human recombinant Interleukin 1 alpha | 0.00001 |
| Carbopol 956 | 2 |
| Menthol | 0.2 |
| Glycerol | 10 |
| Sorbitol | 10 |
| Flavor | 1 |
| Phosphate buffer | qs to pH 5.5 |
| Distilled water | to 100 |

The abovementioned ingredients were mixed in the conventional manner to prepare gel composition containing human recombinant interleukin-1 alpha.

### Example 4

Mouthrinse containing human recombinant interleukin-1 alpha as active ingredient

| Ingredient | Content, wt.% |
|---|---|
| Human recombinant Interleukin 1 alpha | 0.00001 |
| Carbopol 956 | 0.2 |
| Glycerol | 10 |
| Sorbitol | 10 |
| Menthol | 0.2 |
| Phosphate buffer | qs to pH 5.5 |
| Distilled water | to 100 |

The abovementioned ingredients were mixed in the conventional manner to prepare mouthrinse containing human recombinant interleukin-1 alpha.

### Example 5

This example demonstrates the efficacy of toothpaste comprising human recombinant interleukin-1 alpha for reducing oral malodor and gum bleeding.
Eight male subjects with gingivitis applied one gram of the toothpaste of Example 1 (test, n=4) or vehicle (control, n=4) on surface of oral cavity twice-a-day for 1 week. At the 14^{th} day, the average oral odor intensity in the test group was 0.50 scores (not detectable or barely detectable odor) vs. 2.75 (slight to definitely detectable odor) in the control group. Odor intensity was detected by Rosenberg's organoleptic scale. Rosenberg M, et al. J Dent Res, 1991, 70: 1436-40). The bleeding of the gum, the sign of gingivitis, was decreased in test group, while was unchanged in control group. This example shows that oral care composition comprising human recombinant interleukin-1 alpha is useful for reducing oral malodor and gum bleeding in subjects with gingivitis.

## Claims

1. An oral care composition comprising a human recombinant interleukin-1 and an orally acceptable carrier for use in reducing oral malodor in a subject in need thereof.

2. The oral care composition for use according to claim 1, wherein the human recombinant interleukin-1 is human recombinant interleukin-1 alpha or human recombinant interleukin-1 beta.

3. The oral care composition for use according to claim 1, wherein the content of the human recombinant interleukin-1 in said composition is from 10⁻⁷ to 10⁻⁴ wt. %.

4. An oral care composition comprising a human recombinant interleukin-1 and an orally acceptable carrier for use in the treatment or prevention of a periodontal disease.

5. An oral care composition for use according to claim 4, wherein the human recombinant interleukin-1 is human recombinant interleukin-1 alpha or human recombinant interleukin-1 beta.

6. The oral care composition for use according to claim 4, wherein the content of the human recombinant interleukin-1 in said composition is from 10⁻⁷ to 10⁻⁴ wt. %.

7. The oral care composition for use according to claim 4, wherein the periodontal disease is gingival disease.

## Patentansprüche

1. Mundpflegezusammensetzung, umfassend ein humanes rekombinantes Interleukin-1 und einen oral verträglichen Träger zur Verwendung zur Verringerung von Mundgeruch bei einer Person, die dies benötigt.

2. Mundpflegezusammensetzung nach Anspruch 1, worin das humane rekombinante Interleukin-1 ein humanes rekombinantes Interleukinl-alpha oder humanes rekombinantes Interleukin1-beta ist.

3. Mundpflegezusammensetzung nach Anspruch 1, worin der Gehalt des humanen rekombinanten Interleukin-1 in der Zusammensetzung von 10⁻⁷ bis 10⁻⁴ Gew.-% ist.

4. Mundpflegezusammensetzung, umfassend ein humanes rekombinantes Interleukin-1 und einen oral verträglichen Träger zur Verwendung bei der Behandlung oder Verhinderung einer Parodontalerkrankung.

5. Mundpflegezusammensetzung zur Verwendung nach Anspruch 4, worin das humane rekombinante Interleukin-1 ein humanes rekombinantes Interleukinl-alpha oder humanes rekombinantes Interleukin1-beta ist.

6. Mundpflegezusammensetzung zur Verwendung nach Anspruch 4, worin der Gehalt des humanen rekombinanten Interleukin-1 in der Zusammensetzung von 10⁻⁷ bis 10⁻⁴ Gew.-% ist.

7. Orale Zusammensetzung zur Verwendung nach Anspruch 4, worin die Parodontalerkrankung eine Zahnfleischerkrankung ist.

## Revendications

1. Composition de soin buccal comprenant une interleukine-1 humaine recombinante et un support acceptable pour une administration orale pour une utilisation dans la réduction de la mauvaise haleine chez un sujet qui en a besoin.

2. Composition de soin buccal pour une utilisation selon la revendication 1, dans laquelle l'interleukine-1 humaine recombinante est l'interleukine-1 humaine recombinante alpha ou l'interleukine-1 humaine recombinante bêta.

3. Composition de soin buccal pour une utilisation selon la revendication 1, dans laquelle la teneur de l'interleukine-1 humaine recombinante dans ladite composition est de 10⁻⁷ à 10⁻⁴ % en poids.

4. Composition de soin buccal comprenant une interleukine-1 humaine recombinante et un support acceptable pour une administration orale pour une utilisation dans le traitement ou la prévention d'une parodontopathie.

5. Composition de soin buccal pour une utilisation selon la revendication 4, dans laquelle l'interleukine-1 humaine recombinante est l'interleukine-1 humaine recombinante alpha ou l'interleukine-1 humaine recombinante bêta.

6. Composition de soin buccal pour une utilisation selon la revendication 4, dans laquelle la teneur de l'interleukine-1 humaine recombinante dans ladite composition est de 10⁻⁷ à 10⁻⁴ % en poids.

7. Composition de soin buccal pour une utilisation selon la revendication 4, dans laquelle la parodontopathie est une affection des gencives.
